# EUROPEAN PATENT APPLICATION

(11) **EP 2 933 263 A1**
(43) Date of publication of application: **21.10.2015**
(21) Application number: 14165102.6
(22) Date of filing: 17.04.2014
(51) Int. Cl.: C07K 14/245, C12N 15/62, C12N 15/70

(54) **Vector and method for expressing molecules of interest in a bacterial cell**

(71) Applicant: UNIVERSITÄT ZU KÖLN, 50923 Köln (DE)
(72) Inventor: Chmielewski, Markus, 50739 Köln (DE); Abken, Hinrich, 56414 Meudt (DE)
(74) Representative: Kröncke, Rolf

(57) **Abstract**

The present invention relates in a first aspect to a vector nucleic acid suitable for expressing at least one molecule of interest in a prokaryotic cell, in particular a bacterial cell, or on the surface of a prokaryotic cell or released by the prokaryotic cell. In particular, the present invention relates to a vector nucleic acid comprising a nucleic acid sequence encoding a Fim polypeptide. In addition, the present invention relates to a cell containing said vector as well as isolated recombinant protein containing at least a module being a Fim polypeptide and a module being a molecule of interest. In addition, the present invention relates to methods for the production of the molecule of interest using the vector nucleic acid according to the present invention. Finally, the present invention provides a kit of system containing said vector nucleic acid in particular for the production of recombinant molecule of interest.

SEQUENCE LISTING

<110>  Universitaet zu Koeln

<120>  Vector and method for expressing molecules of interest in a
       bacterial cell

<130>  4677-004 EP-1

<160>  2

<170>  PatentIn version 3.5

<210>  1
<211>  21
<212>  PRT
<213>  Escherichia coli

<400>  1

Met Val Arg Val Ile Thr Leu Phe Ala Val Leu Leu Met Gly Trp Ser
1               5                   10                  15

Val Asn Ala Trp Ser
            20

<210>  2
<211>  279
<212>  PRT
<213>  Escherichia coli

<400>  2

Phe Ala Cys Lys Thr Ala Asn Gly Thr Ala Ile Pro Ile Gly Gly Gly
1               5                   10                  15

Ser Ala Asn Val Tyr Val Asn Leu Ala Pro Val Val Asn Val Gly Gln
            20                  25                  30

Asn Leu Val Val Asp Leu Ser Thr Gln Ile Phe Cys His Asn Asp Tyr
        35                  40                  45

Pro Glu Thr Ile Thr Asp Tyr Val Thr Leu Gln Arg Gly Ser Ala Tyr
    50                  55                  60

Gly Gly Val Leu Ser Asn Phe Ser Gly Thr Val Lys Tyr Ser Gly Ser
65                  70                  75                  80

Ser Tyr Pro Phe Pro Thr Thr Ser Glu Thr Pro Arg Val Val Tyr Asn
                85                  90                  95

Ser Arg Thr Asp Lys Pro Trp Pro Val Ala Leu Tyr Leu Thr Pro Val
            100                 105                 110

Ser Ser Ala Gly Gly Val Ala Ile Lys Ala Gly Ser Leu Ile Ala Val
        115                 120                 125

Leu Ile Leu Arg Gln Thr Asn Asn Tyr Asn Ser Asp Asp Phe Gln Phe
    130                 135                 140

Val Trp Asn Ile Tyr Ala Asn Asn Asp Val Val Val Pro Thr Gly Gly
145                 150                 155                 160

Cys Asp Val Ser Ala Arg Asp Val Thr Val Thr Leu Pro Asp Tyr Pro
                165                 170                 175

Gly Ser Val Pro Ile Pro Leu Thr Val Tyr Cys Ala Lys Ser Gln Asn
            180                 185                 190

Leu Gly Tyr Tyr Leu Ser Gly Thr Thr Ala Asp Ala Gly Asn Ser Ile
        195                 200                 205

Phe Thr Asn Thr Ala Ser Phe Ser Pro Ala Gln Gly Val Gly Val Gln
    210                 215                 220

Leu Thr Arg Asn Gly Thr Ile Ile Pro Ala Asn Asn Thr Val Ser Leu
225                 230                 235                 240

Gly Ala Val Gly Thr Ser Ala Val Ser Leu Gly Leu Thr Ala Asn Tyr
                245                 250                 255

Ala Arg Thr Gly Gly Gln Val Thr Ala Gly Asn Val Gln Ser Ile Ile
            260                 265                 270

Gly Val Thr Phe Val Tyr Gln
        275

## Description

The present invention relates in a first aspect to a vector nucleic acid suitable for expressing at least one molecule of interest in a prokaryotic cell, in particular bacteria, or on the surface of and released by said cell. In particular, the present invention relates to a vector nucleic acid comprising a nucleic acid sequence encoding a Fim polypeptide fused with a molecule of interest. In addition, the present invention relates to a cell containing said vector as well as isolated recombinant protein containing at least one module being a Fim polypeptide and a further module being a molecule of interest. In addition, the present invention relates to methods for the production of the molecule of interest using the vector nucleic acid according to the present invention. Finally, the present invention provides a kit of system containing said vector nucleic acid in particular for the production of recombinant molecule of interest.

### Prior art

The expression and production of recombinant proteins in bacterial cells is a long standing and well known procedure for obtaining recombinant proteins. A preferred host cell is the *Escherichia coli* (E. coli) bacterium. An expression system is a combination of a vector, namely an expression vector, and a host whereby said host allows the expression of the gene present in the expression vector in the host. The expression can be permanent or may be regulated by induction allowing transient or switching on and off of the expression accordingly. Expression systems are used for the expression of recombinant proteins starting from research to the industrial scale to produce reagents suitable for diagnostics and therapy.

Various expression systems are known in the art depending on the desired purpose and the host cell. Most often procaryotic cells, mostly bacteria are used as host cells since they can be easily genetically manipulated, however, also eukaryotic expression systems for use in plants, insects and mammalian cells are described and established. Typically, the expression vector comprises a multiple cloning site allowing the incorporation of the desired molecule to being expressed, also identified as the molecule of interest (MOI). In addition, the expression vector contains suitable promoter sequences allowing transcription of the nucleic acid sequence present in the vector system. The expression vector may be an expression vector allowing for extrachromosomal expression or may be an expression vector allowing integration of the nucleic acid sequence to be expressed into the genome of the host.

The expression vector is obtained by genetic engineering and transformation of the bacteria is effected by various methods.

Also the production of recombinant proteins in expression systems using bacterial cells as host cells is well known in the art, the process is limited for various reasons. For example, expression in bacterial systems is limited due to cytoplasmic or periplasmic accumulation of the protein of interest, e.g. in inclusion bodies. Actually, one of the main disadvantages of the bacterial expression system is the accumulation of the expression product in inclusion bodies, thus, the dissolution of the desired expression product is not given. Various attempts have been made in the art to overcome the problem of accumulation of the expression product in inclusion bodies and, in addition, allowing secretion of the expression product in the culture medium. For review: Pines O, Inouye M. Expression and secretion of proteins in E. coli., Mol Biotechnol. 1999 Aug;12(1):25-34.

To increase solubility of the recombinant expression products, it is tried to overcome the formation of inclusion bodies, e.g. using expression systems containing GST (Glutathione-S-Transferase) or N utilizations substance A (NusA). Alternatively, it is tried to direct the expression proteins into the periplasma. However, expressions systems allowing direct expression and equitation of the expression product into the extracellular environment are seldom.

Expression systems for proteins of interest in gram-negative bacteria are so far based on C-terminal fusion or integration within the bacterial carrier protein. In E. coli, the outer membrane OmpA, LamB, PhoE have been used to display peptides or proteins on the cell surface (Etz, et al., J Bacteriol. 183:6924-35, 2001). In Salmonella, the Omp bacterial transporter was used to express a protein of interest (Massa, et al., Salmonella engineered to express CD20-targeting antibodies and a drug-converting enzyme can eradicate human lymphomas Blood 2013 122:705-714). However, insertion of peptides longer than 60 amino acids was shown to perturb the conformation of LamB and PhoE (Agterberg, et al, Gene 88:37-45, 1990; Charbit et al., Gene 70:181-9, 1988), resulting in interference with proper cell surface localization. The OmpA expression system is additionally limited in the use of hydrophobic segments or inserts with distinct secondary structures (Hobom et al., Dev Biol Stand. 84:255-62, 1995).

So far, the fimbrial protein FimH has been used to display peptides incorporated within the bacterial carrier protein and thereby limited the potential size of the integrated DNA. FimH belongs to the group of adhesin proteins.

Adhesins are cell surface components or appendages of bacteria that facilitate bacterial adhesion or adherence to other cells or to inanimate surfaces. Adhesion represents an essential step in bacterial pathogenesis or infection required for colonising a new host. In gram-negative bacteria fimbria function as adhesion structures. That is, to effectively achieve adherence to host surfaces, many bacteria produce multiple adherence factors called adhesins. A typical structure of the bacteria adhesion is that of fimbria. In contrast to pili, fimbria are not involved in the exchange of genetic material, however, fimbria are often called type-1-pili. Gram-negative pathogens commonly interact with their environment using long, linear, surface-exposed protein appendages. In uropathogenic Escherichia coli, type-1 pili carry at their distal end a dedicated mannose-specific adhesin, Fim H, that is responsible for the attachment of bacteria to the bladder epithelium and their subsequent internalization and biofilm-like organization inside the urothelial cells. Type-1 pili are representative of a large class of non-covalently linked fibres on the surface of gram-negative bacteria, synthesized via the conserved chaperone/usher pathway. Type-1 pili are composed of four different subunit types (FimH, FimG, FimF and FimA).

In Gram-negative bacteria many adhesins are displayed on the bacterial surface by chaperone-usher-assisted transport, an illustrative example being type-1 pili. A typical type-1 fimbriated bacterium has 200-500 peritrichously arranged pili on the surface. A single pilus is composed of four building elements that are added to the base of a growing organelle. Approximately 1.000 copies of the major structural component, FimA, are polymerized into a righthanded helical structure, which additionally contains small quantities of the minor components, FimF, FimG, and FimH. Hence, type-1 pili produced by gram-negative bacteria like E.coli are multi-subunit fibres crucial in the recognition of and the adhesion to host cells. Type 1 fimbria are composed of four different subunit types (Fim H, Fim G, Fim F and Fim A). The ad-hesin Fim H and the two linker subunits Fim G and Fim F form a short flexible fibrillar tip that is attached to an extended rigid and helically wound rod of Fim A subunits.

Translocation of the structural components across the inner membrane is mediated by the Sec-dependent pathway. The minor components, notably Fim H are of paramount importance for the initiation of organelle formation and determines the organelle length and number in a dose-dependent manner.

Fim H is one of the best characterized bacterial adhesin. This adhesin is responsible for D-mannose sensitive adhesion. Mature Fim H is displayed on the bacterial surface as a component of the type-1 pili.

Fim H is involved in modulating the immune response against pathogens. For example Fim H overcomes the antibody based immune response by natural conversion from the high to the low affinity state. Through this conversion, Fim H adhesion may shed the antibodies bound to it. Vaccines were based on adhesins because they are often essential to infection and are surface located, making them readily accessable to antibodies.

Taken together, Fim H is of interest for biotechnological production of proteins. Fim H adhesion proteins and methods of use are described, e.g. Fim H adhesion proteins and methods of use are described e.g. in WO 02/04496. Therein the bacterial adhesin proteins and active fragments thereof, in particular, for use in vaccine composition are disclosed.

Pallesen et. al., Microbiology, 1995, 141, 2839-2848, describe a chimeric FimH adhesion type-1 pili useful as a bacterial surface display system for heterologous sequences. Therein heterologous sequences are incorporated into the FimH protein positioned in the C-terminal domain of FimH. It is identified therein, that the C-terminal domain of FimH is amenable for integration of heterologous inserts of substantial size. Further, it is speculated therein that the chimeric FimH proteins can be used for presentation of immunologic relevant epitopes. Moreover other possibilities include protein-protein interaction and receptor recognition in general. Of note, this document identifies integrated expression of the heterologous sequence, however, expression of a protein of interest on the bacteria surface or purification of the heterologous products is not envisaged. In Asadi Karam MR et.al., Iranian J Microbiology, 2012, 4(2), 55-62, cloning of FimH and fliC and expression thereof as fusion protein is described. However, the fusion protein in the described fashion is not suitable for expression on the surface of the bacteria or for secretion into the culture medium.

### Brief summary of the invention

In a first aspect, the present invention provides a vector nucleic acid suitable for expressing at least one molecule of interest (MOI) in a prokaryotic cell or on the surface of a prokaryotic cell, in particular bacteria, comprising in frame form 5' to 3' a nucleic acid encoding a leader sequence, a multiple cloning site for introduction of the nucleic acid sequence encoding said MOI and a nucleic acid sequence encoding a mature Fim polypeptide.

In a further aspect, the present invention provides a vector nucleic acid suitable for expressing at least one molecule of interest in a procaryotic cell or in the surface of the said cell comprising a nucleic acid sequence encoding in frame from 5' to 3' a nucleic acid encoding a leader sequence, a nucleic acid sequence encoding a molecule of interest and a nucleic acid sequence encoding a Fim polypeptide, optionally having a linker nucleic acid sequence located between the nucleic acid sequence encoding a molecule of interest and the nucleic acid sequence encoding a mature Fim polypeptide. Said optionally present linker is a linker which does not change the reading frame of the nucleic acid sequence.

Further, the present invention provides a cell containing the vector nucleic acid according to the present invention. The cell contains said vector nucleic acid according to the present invention either stably integrated in the host or present as a minicircle expression vector for a stable or transient expression.

Moreover, an isolated recombinant protein containing at least a first module being a Fim polypeptide and a second module being a molecule of interest, like a protein of interest, is provided.

In addition, the present invention provides a method for production of a molecule of interest using the vector nucleic acid according to the present invention for transforming a prokaryotic cell.

In addition, the present invention provides a method for screening for a molecule of interest using the vector nucleic acid according to the present invention for displaying the molecule of interest on the surface of a prokaryotic cell and using that transformed cell for screening.

Finally, the present invention relates to a kit or system containing the vector nucleic acid according to the present invention, in particular, for use in the production of recombinant molecules.

### Brief description of the drawings

Fig. 1: Figure 1 is a schematic model of the synthesis and transport of the fusion protein GlucFimH in bacterial cells. Shown is the pilus where the fusion protein is present on the outer surface of the bacteria. The fusion protein is eventually released into the extracellular space.
Fig. 2: Figure 2 shows the structure of a vector nucleic acid according to the present invention.
Fig.3: Figure 3 is an analysis of transformed bacteria cells. Shown are the flow cytometry data of wild type bacteria of transformed bacteria which express the fusion protein on the surface.
Fig. 4: Figure 4 shows the luciferase light signals of modified bacteria *in vitro* and in a pancreatic cancer mouse model.

### Detailed description of the present invention

In a first aspect, the present invention relates to a vector nucleic acid suitable for expressing at least one molecule of interest in a prokaryotic cell or on the surface of that cell or release of the product by that cell in frame from 5' to 3' a nucleic acid encoding a leader sequence, a multiple cloning site for introduction of the nucleic acid sequence encoding said MOI and a nucleic acid sequence encoding a mature Fim polypeptide.

In this connection, the term "comprise" and "comprising" as well as "contain" and "containing" includes the embodiments of "consist" and "consisting of". That is, the terms "comprising" and "comprises" and "comprised of" are synonymous with "including", "includes" or "containing", "contains", and are inclusive or open ended and do not exclude additional, non recited members, elements or method steps.

As used herein, the singular forms "a", "an" and "the" include both singular and plural reference unless context clear dictates otherwise.

Unless otherwise defined, all terms used in disclosing the invention, including technical and scientific terms, have the meaning as commonly understood by one of the ordinary skilled in the art to which this invention belongs. By means of further guidance, term definitions are included to better appreciate the teaching of the present invention.

All references cited in the present application are hereby incorporated by reference in their entirety. In particular, the teachings of all references herein specially referred to an incorporated by reference.

The term "vector nucleic acid" refers to a nucleic acid sequence present in form of a vector, typically, an expression vector which is usually a plasmid designed for expression in host cells, in particular, bacterial cells. The vector is used to introduce a specific gene into a host cell and can allow protein synthesis based on the specific gene introduced to produce a recombinant protein encoded by said gene. The vector nucleic acid is an engineered vector containing regulatory sequences. Said regulatory sequences can include enhancer and promoter regions allowing a transient or induced transcription of the gene. Said vector nucleic acid is designed to be transformed or transfected into the host cell for protein synthesis. Said vector nucleic acid may be designed to have elements allowing insertion of the nucleic acid sequence into the host chromosome. Alternatively, said vector nucleic acid may contain elements allowing episomal replication. The skilled person is well aware of vector nucleic acid sequences suitable according to present invention.

The vector nucleic acid sequence according to the present invention contains a nucleic acid sequence encoding a Fim polypeptide. Said nucleic acid sequence encoding the Fim polypeptide contains a multiple cloning site for introduction of the nucleic acid sequence encoding the molecule of interest (MOI) 5' to the reading frame of the mature Fim polypeptide. That is, in an embodiment, the multiple cloning site (MCS) is located in frame between the nucleic acid encoding a leader sequence and the nucleic acid sequence encoding the mature polypeptide.

As used herein, the term "Fim polypeptide" refers to a Fim protein described in the art derived from bacterial cells, in particular, stemming from E.coli. For example, the Fim polypeptide is a Fim H, Fim G, or Fim A protein. The term "Fim polypeptide" includes embodiments wherein the Fim polypeptides are equivalents of the naturally occurring Fim proteins having the same functionality, eg. fragments of said polypeptides. In addition, homologues of the Fim polypeptide may be used referring to polypeptides having the same functionality but being obtained from other natural sources or being obtained recombinantly or synthetically.

As used herein, the term "leader sequence" refers to a sequence directing the molecule composed of the MOI and the mature Fim polypeptide to a predetermined compartment of the host cell, e.g. in type-1 pili on the bacterial surface. In an embodiment of the present invention, the leader sequence is the Fim leader sequence, e.g. the Fim H leader sequence.

Further, as used herein, the term "mature" refers to a polypeptide not containing any leader sequence. The term " nucleic acid sequence encoding a mature Fim polypeptide" refers to a sequence without the Fim leader or signal sequence.

As used herein, the term "molecule of interest (MOI)" refers to molecules, either on nucleic acid level, also referred to as gene of interest, GOI, or amino acid level, also referred to as POI, protein of interest. In particular, the MOI is a (poly-)peptide or protein to be expressed in the host cell.

The present inventors recognized that the Fim polypeptide represents a suitable means for promoting expression and delivery of the MOI expressed in a prokaryotic cell, in particular bacteria, to the surface of said bacterial cell or allowing release from the bacterial cell, thus, overcoming the disadvantage known in the art, like aggregation of the recombinantly expressed MOI in inclusion bodies.

Hence, another embodiment of the present invention relates to a vector nucleic acid symbol for expressing at least one molecule of interest in a prokaryotic cell or on the surface of the said cell in frame from 5' to 3' a nucleic acid encoding a leader sequence, a nucleic acid sequence encoding a molecule of interest and a nucleic acid sequence encoding a mature Fim polypeptide, optionally, having a linker nucleic acid sequence located between the nucleic acid sequence encoding a molecule of interest and the nucleic acid sequence encoding a mature Fim polypeptide. For example, the linker is at least partly composed of parts of the multiple cloning sites. In addition, the linker nucleic acid sequence may encode elements or means allowing separating the expressed molecule of interest from the Fim moiety of the expressed fusion protein. The vector nucleic acid according to present invention allows the expression of a fusion protein comprising the Fim polypeptide moiety and a moiety of the molecule of interest, optionally, separated by a linker moiety. Typically, the molecule of interest is a protein or peptide.

In an embodiment of the present invention, the vector nucleic acid includes a linker nucleic acid sequence present in frame between the nucleic acid sequence encoding the molecule of interest the nucleic acid sequence encodingand the mature Fim polypeptide. For example, the linker nucleic acid sequence encodes a cleavage site allowing separation of the Fim polypeptide and the molecule of interest after expression. Alternatively, the linker nucleic acid sequence may be arranged at the 5' end of the nucleic acid sequence allowing purification of the fusion protein of the Fim polypeptide and the molecule of interest. The skilled person is well aware of the order of elements present in the vector nucleic acid. Usually, the order is given from the 5' to the 3' of the nucleic acid sequence. That is, the nucleic acid sequence encoding the molecule of interest is usually located upstream of the mature Fim nucleic acid sequence and downstream of the leader sequence. The multiple cloning site is at the 5' end of the nucleic acid sequence encoding the mature Fim polypeptide. In an embodiment, the order for 5' to 3' is a nuclei acid encoding a leader sequence, MOI, optionall a linker, and the mature FimH. Further, a tag may be present allowing purification of the fusion protein after expression or of the MOI after expression.

In an embodiment of the present invention, the vector nucleic acid contain further a nucleic acid sequence operable linked with the nuclear acid sequence encoding the Fim polypeptide and the nuclear acid sequence encoding the molecule of interest. Said nuclear acid sequence encodes a tag molecule. The tag molecule may be located at the 5' end or 3' end of the vector nucleic acid and, accordingly, the N-terminal end and/or C-terminal end of the polypeptide. In some embodiments, the tag moiety is present downstream of the nucleic acid sequence encoding the Fim polypeptide and the nucleic acid sequence encoding the molecule of interest.

The skilled person is well aware of suitable tag molecules including peptide tags like FLAG-tag, HA-tag, His-tag, Myc-tag, S-tag SBP-tag, Softag 1, Softag 3, TC tag, V5 tag, Xpress tag.

In an embodiment, the leader sequence is a leader sequence of a Fim Protein, e.g. of the Fim H protein.

In a further embodiment, the nucleic acid sequence according to the present invention comprise further elements including at the 5' end of said nucleic acid sequence an inducible promoter operably linked with leader sequence. Said inducible promoter allows transient expression of the nucleic acid comprising the coding sequence for Fim and the MOI.

The skilled person is well aware of suitable promoter sequence being inducible, in particular, being inducible in bacterial cells. For example, the promoter of the vector nucleic acid may be arrived from any known promoter/enhancer system described in the art. Preferably, the promoter/enhancer region is derived from the T7 RNA polymerase promoter to direct high-level expression of the Fim polypeptide and the molecule of interest.

In addition, the vector nucleic acid sequence contains further elements for expression, for example a Shine-Dalgarno-sequence. The promoter may be derived from the LAC operator or the T7 promoter for example regulated by the LAC operator. The skilled person is well aware of suitable promoter and enhancer systems as well as of suitable regulators of the expression in bacterial cells. Furthermore, the vector nucleic acid contains termination elements as described in the art.

If required, suitable selectable markers are present in the vector nucleic acid as well.

That is, in an embodiment of the present invention, the vector nucleic acid is a vector nucleic acid in the order from the 5' end to the 3' end of a 5'-promoter, a nucleic acid encoding a leader sequence, a nucleic acid sequence encoding the molecule of interest, optionally a linker sequence, the nucleic acid sequence encoding a Fim polypeptide and a transcription termination sequence and translation termination sequence.

For example, the nucleic acid sequence encoding the leader sequence is a sequence encoding an amino acid sequence according to Seq. ID No. 1. Further, the nucleic acid encoding the mature Fim polypeptide is a nucleic acid sequence encoding the amino acid sequence according to Seq. ID No.2. The nucleic acid sequence encoding the molecule of interest is a nucleic acid sequence encoding a polypeptide, peptides or proteins of mammalian origin including human.

In another embodiment, the vector nucleic acid is a vector nucleic acid wherein the molecule of interest being secreted extracellularly or being expressed on the surface of the bacterial cell and having biological activity. The fusion protein of Fim and the molecule of interest is located on the surface of the bacterial cells, thus, the fusion protein may be isolated from the surface of the bacterial cells or, alternatively, be obtained from the supernatant of the bacterial culture wherein said fusion proteins are sheded or secreted. In an embodiment, the fusion protein of Fim and the MOI or the MOI may be sheded or cleaved into the supernatant based on appropriate cleavage sites present in linker moieties. The skilled person is well aware of suitable systems allowing cleavage between the Fim and the MOI or cleavage of Fim and MOI accordingly.

The Fim polypeptide encoded in the vector nucleic acid according to present invention may be selected from Fim H, Fim A, Fim F and/or Fim G. In a preferred embodiment, the Fim polypeptide is Fim H. The leader sequence may be a sequence derived from Fim H, Fim A, Fim F and/or Fim G. In a preferred embodiment, the Fim polypeptide is Fim H.

In another embodiment, the present invention relates to a bacterial cell containing a vector nucleic acid according to the present invention wherein said vector nucleic acid may be stably integrated into the host genome or alternatively, or additionally, may be present as plasmids in the bacterial cell. The cell may stably express the polypeptide encoded by the vector nucleic acid or may transiently express the same.

For example, in case of transient expression, the vector nucleic acid contains an inducible promoter allowing transient expression accordingly.

The vector nucleic acid may be introduced into the cells, i.e. the bacterial cells may be transformed with the vector nucleic acid, by known methods.

In a further aspect, the invention relates to the isolated recombinant protein containing at least one module being a Fim polypeptide and a second module being a molecule of interest whereby the MOI being at the N-terminus of the mature Fim polypeptide.

"Isolated" in the context of the present invention with respect to polypeptides means that the material is removed from its original environment like the cells used to recombinantly produce the polypeptides disclosed herein. These peptides could be part of a composition and still be isolated in that such vector or composition is not part of its natural environment. The polypeptides or protein according to the present invention are preferably provided in an isolated form, and preferably are purified to homogeneity.

The terms polypeptide, peptide and protein are used herein interchangeably unless otherwise identified.

In an embodiment, the isolated recombinant protein according to present invention is obtainable by expression thereof using the vector nucleic acid molecule according to the present invention. That is, the isolated recombinant protein is an expression product of the vector nucleic acid according to the present invention. The term "expression product" means that the polypeptide or protein is the natural translation product of the gene and any nucleic acid sequence coding equivalents resulting from genetic code degeneracy and, thus, coding for the same amino acid(s).

It is clear to the skilled person that the nucleic acid sequence encoding the Fim polypeptide and/or the nucleic acid sequence encoding the molecule of interest include coding equivalents from genetic code degeneracy but encoding for the same amino acids. This is particularly true for the MOI where the nucleic acid sequence encoding the MOI may be adapted to the genetic code of the bacterial cell wherein said nucleic acid sequence is used and is translated into the peptide accordingly.

In an embodiment of the present invention, the nucleic acid sequence encoding the Fim polypeptide is obtained from the bacterial species used as the host for protein expression using the vector nucleic acid according to the present invention. Other embodiments include Fim polypeptides and nucleic acid sequences encoding said Fim polypeptides wherein the Fim is originated from other bacterial species.

Furthermore, the present invention relates to a method for the production of the molecule of interest. Said method comprises a step of transforming a prokaryotic host cell, in particular a bacterial cell, with a vector nucleic acid according to the present invention or using a cell containing the vector according to the present invention.

In an embodiment of said method for the production of the MOI, said method comprises further the step of introducing the nucleic acid sequence encoding the MOI into the vector nucleic acid according to the present invention comprising a nucleic acid sequence encoding a Fim polypeptide followed by a multiple cloning site suitable for introduction of the nucleic acid sequence encoding a molecule of interest within the reading frame of the Fim polypeptide. Said step is conducted before transforming the bacterial cell with said vector nucleic acid. The skilled person is well aware of suitable methods and means for introducing said nucleic acid sequence including the molecule of interest into the multiple cloning site of the vector nucleic acid whereby the nucleic acid sequence encoding the MOI is in the reading frame of the Fim polypeptide.

In an embodiment of said method for the production of the MOI, the recombinant protein produced in the host cell, namely, the bacterial cell is released by or expressed on the surface of said transformed host cell.

The fusion protein according to present invention comprising the Fim moiety and the MOI moiety, for example in form of the Fim H and the MOI moiety are part of the Typ-I pili on the surface of the bacterial cell. Such modified bacteria can be used for screening for a MOI with particular properties, e.g. for binding or enzymatic activities. In such an embodiment, transformed bacteria are incubated on surfaces or beads coated with the ligand for the MOI using standard binding techniques ("display" or "panning") and bound bacteria are analysed with respect to the Fim H fusion protein to identify the MOI. In case of enzymatic activities which are characteristic for the MOI, transformed bacteria are incubated on plates or membranes coated with the substrate and the Fim H fusion protein of bacteria colonies which metabolize the substrate are further analysed with respect to the MOI.

In a further embodiment, the method for the production of the MOI comprises further steps of culturing the transformed bacterial cells in a culture medium, separating the bacterial cells by culture on the selective culture medium and purifying the MOI from the cell free culture medium. Alternatively or additionally, the bacterial cells are treated by known means to separate the fusion protein or the MOI from the surface of the bacterial cells.

Finally, the present invention provides a kit or system containing the vector nucleic acid according to the present invention, or a cell according to the present invention. The kit or system according to the present invention is particularly useful for the production of recombinant molecules of interest, in particular, molecules of interest having a natural folding structure and the same biological activity of the natural molecule of interest.

The kit or system according to the present invention may contain additionally prokaryotic cells, preferentially bacterial cells, to be used for transformation. Further, the kit or system may contain suitable reagents including buffer and culture medium for transforming the said host cells and for culturing the same.

Moreover, the kit or system may contain additionally means for allowing separation and isolation of the fusion protein or the MOI alone, e.g. based on the tag moiety present in the recombinant protein.

A main advantage of the MOI, typically in form of a polypeptide or protein, also identified as a protein of interest or polypeptide of interest (POI), is it's natural folding structure. In addition, the biological activity of the MOI, e.g. in form of the POI, is more or less identical with the biological activity of the natural molecule of interest. So far, no systems are described allowing expression of MOI on the surface of bacterial cells or the production of secreted MOI having a natural folding structure and the same biological activity. The present invention overcomes the problems known in the art in view of enrichment in the periplasm of the bacterial cells or in the inclusion bodies of the bacterial cells or similar procedures.

In an embodiment of the present invention, the MOI is a toxin, in particular, a cytotoxic component. For example, the MOI is a moiety being cytotoxic to mammalian cells, in particular, tumour cells.

### Examples

### Expression of the FimH-GaussiaLuc fusion protein on the surface of BL21-Gold (DE3) E.coli.

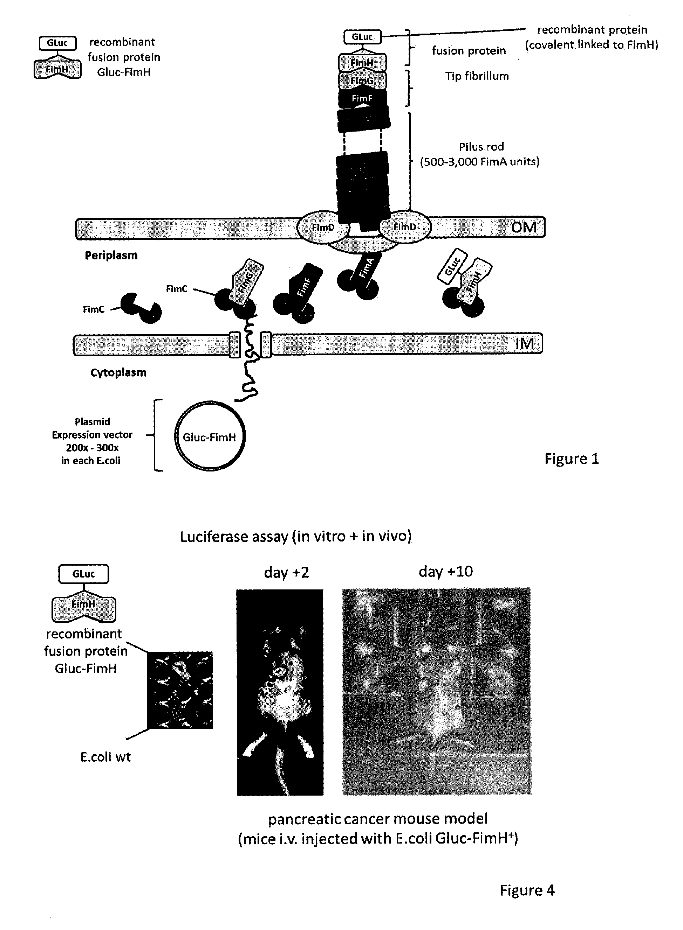

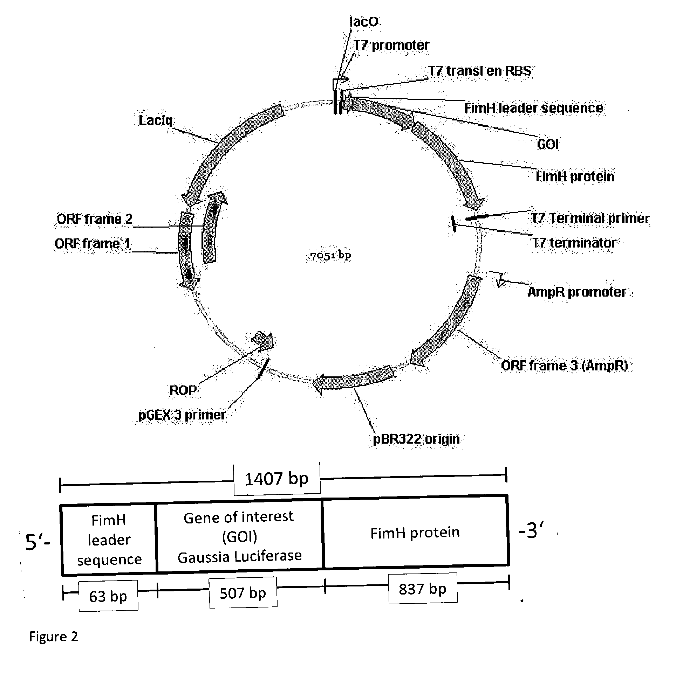

To produce the MOI GaussiaLuc, the cDNA coding for leader FimH-GaussiaLuc-FimH (see Fig.2) was amplified by PCR (polymerase chain reaction) and cloned into the T7 expression vector pET-11d (Agilent Technologies, Stratagene Products Division, La Jolla, CA 92037, US) using the restriction sites Ncol and BamHl. The pET expression vector, derived from the pBR322 plasmid, is engineered to take advantage of the featuers of the T7 bacteriophage gene 10 that promotes high-level transcription and translation. The integration of the lac operator between the T7 promoter and translation initiation sequences allows IPTG-mediated de-repression of the T7 promoter in addition to IPTG-induction of T7 polymerase from *IacUV5* promoter in the DE3 containing bacterial strains (*E.coli* BL21-derived high expression strains). The BL21 strain is generally suitable for protein expression due to its deficiency in *Ion* protease as well as *ompT* outer membrane protease that can degrade proteins. The originally gene 10 leader sequence was replaced by FimH leader sequence due to the intended destination of the expressed protein of interest on the bacterial surface. Fig. 1 shows a model of the synthesis of the Gluc-FimH expression in bacterial cells.

### Inducible Expression of the p-ET11 d-GaussiaLuc-FimH vector on the bacterial surface.

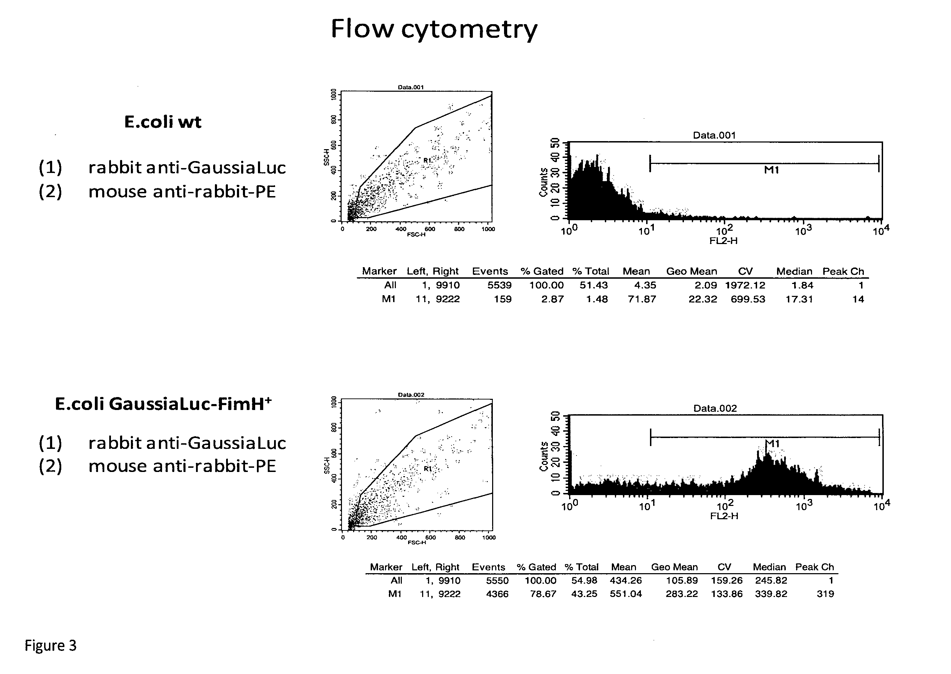

The BL21-Gold expression strain BL21-Gold (DE3) pLysS (Agilent technologies) was transformed with the p-ET11 d-GaussiaLuc-FimH vector. Expression of GaussiaLuc-FimH was induced by IGPT administration and analysed by flow cytometry. Therefore, BL21-Gold E.coli with GaussiaLuc-FimH expression were co-incubated with GaussiaLuc-specific rabbit serum [1µg/ml] and with the PE-conjugated rabbit IgG1-specific antibody. Non-transformed BL21-Gold E.coli served as control. Bacteria transformed with the p-ET11d-GaussiaLuc-FimH vector express the GaussiaLuc protein on the surface as detected by a specific antibody. Fig.3.

To demonstrate that the GaussiaLuc protein is functional, transformed bacteria were incubated with benzyl-coelenterazine, which is a substrate for Gaussia luciferase, and light emission was recorded using a Biospace imaging device Biospace Lab, Paris). E.coli bacteria transformed with the p-ET11d-GaussiaLuc-FimH vector show light emission which is not the case with non-transformed E.coli bacteria (Fig.4, left side).

To demonstrate activity of the GaussiaLuc protein expressing bacteria in vivo, transformed bacteria were injected into the tail vein of mice which harbor a transplanted pancreatic cancer in the pancreas upon panc02 cell transplantation. Bioluminescence imaging to detect the GaussiaLuc bacteria was performed upon intra-peritoneal injection of benzyl-coelenterazine (100 µg/mouse or 5 µg/ml) (PJK GmbH, Kleinblittersdorf, Germany) as substrate for the GaussiaLuc. Accumulated transformed bacteria were visualized using the Biospace Imager (Biospace Lab, Paris, France). The light signal indicates GaussiaLuc activity at the site of pancreatic cancer and affected lymph nodes in treated mice. Non-modified bacteria did not produce a light signal (Fig. 4, right side).

## Claims

1. A vector nucleic acid suitable for expressing at least one molecule of interest in a prokaryotic cell, in particular bacterial cell, or on the surface of said cell or released by the said cell comprising in frame from 5' to 3' a nucleic acid sequence encoding a leader sequence, a multiple cloning site for introduction of the nucleic acid sequence encoding a molecule of interest and a nucleic acid sequence encoding a mature Fim polypeptide.

2. A vector nucleic acid suitable for expressing at least one molecule of interest in a prokaryotic cell, in particular, a bacterial cell or on the surface of a said cell comprising
a nucleic acid sequence encoding in frame from 5' to 3' a nucleic acid sequence encoding a leader sequence, a nucleic acid sequence encoding a molecule of interest and, a nucleic acid sequence encoding a mature Fim polypeptide, optionally, having a linker nucleic acid sequence located between the nucleic acid sequence encoding a molecule of interest and the nucleic acid sequence encoding a mature Fim polypeptide.

3. The vector nucleic acid according to claim 2 wherein a linker nucleic acid sequence is present in frame between the nucleic acid sequence encoding the molecule of interest and the nucleic acid sequence encoding the mature Fim polypeptide, in particular, wherein the linker nucleic acid sequence encodes a cleavage site allowing separation of the Fim polypeptide and the molecule of interest after expression.

4. The vector nucleic acid according to any one of claims 2 to 3 further containing a nucleic acid sequence operably linked with the nucleic acid sequence encoding the Fim polypeptide and the nucleic acid sequence encoding the molecule of interest whereby said nucleic acid sequence encodes a tag molecule.

5. The nucleic acid sequence according to any one of the preceding claims comprising at the 5' end of said nucleic acid sequence an inducible promoter operably linked with said nucleic acid sequence encoding the leader sequence.

6. The vector nucleic acid according to any one of the preceding claims whereby the molecule of interest being released extracellulary or being expressed on the surface of the prokaryotic cell and having biological activity.

7. The vector nucleic acid according to any one of the preceding claims
wherein the Fim polypeptide is Fim H, A, F, or G, in particular Fim H, or a Fim homologue.

8. The vector nucleic acid according to any one of the preceding claims
wherein the leader sequence is a Fim leader sequence, in particular, a FimH leader sequence.

9. Cell containing a vector nucleic acid as defined in any one of claims 1 to 8 wherein said vector nucleic acid may be i) stably integrated into the host genome or episomally replicating or ii) may be present as a minicircle expression vector for stable or transient expression.

10. Isolated recombinant protein containing at least one module being a mature Fim polypeptide and a second module being a molecule of interest whereby the molecule of interest being located at the N-terminus of the mature Fim polypeptide.

11. The isolated recombinant protein according to claim 10 obtainable by expression thereof using a vector nucleic acid according to any one of claims 1 to 8.

12. A method for the production of a molecule of interest comprising the step of transforming a prokaryotic cell, in particular, a bacteria, with the vector nucleic acid as defined in any one of claims 1 to 8, optionally comprising further the step of introducing the nucleic acid sequence encoding the molecule of interest into the vector nucleic acid according to claim 1 before transforming the prokaryotic cell with said vector nucleic acid.

13. The method for the production of a molecule of interest according to claim 12 for obtaining recombinant protein secreted by or expressed on the surface of the transformed cell.

14. The method for the production of a molecule of interest according to claims 12 or 13 further comprising the steps of culturing the transformed bacterial cells in a culture medium, optionally, separating the intact bacterial cells and the culture medium, and purifying the molecule of interest from the cell free culture medium.

15. A kit or system containing a vector nucleic acid as defined in any one of claims 1 to 8 or a cell according to claim 9.

16. The use of a kit or system according to claim 15 for the production of recombinant molecules of interest, in particular, of molecules of interest having a natural folding structure and the same biological activity as the natural molecule of interest.
